# EUROPÄISCHE PATENTANMELDUNG

(11) **EP 3 202 433 A1**
(43) Veröffentlichungstag der Anmeldung: **09.08.2017**
(21) Anmeldenummer: 16154223.8
(22) Anmeldetag: 04.02.2016
(51) Int. Cl.: A61M 1/10

(54) **AUSLASSGRAFT SOWIE SYSTEM UMFASSEND EINE BLUTPUMPE UND EINEN AUSLASSGRAFT**

(71) Anmelder: Berlin Heart GmbH, 12247 Berlin (DE)
(72) Erfinder: KALLENBACH, Sebastian, 34128 Kassel (DE); PHILLIPS, Daniel, 10249 Berlin (DE)
(74) Vertreter: Pfenning, Meinig & Partner mbB

(57) **Zusammenfassung**

Gegenstand der vorliegenden Anmeldung ist ein Auslassgraft für eine Blutpumpe sowie ein System, das eine Blutpumpe und einen an einen Auslass der Blutpumpe angebundenen Auslassgraft umfasst. Der Auslassgraft umfasst einen ersten, parallel zur Längsachse verlaufenden Streifen, wobei der Streifen aus einem Material besteht, das unter Röntgenstrahlung kontrastreicheren als die Umhüllung ist.

## Beschreibung

Gegenstand der vorliegenden Erfindung ist ein Auslassgraft sowie ein System mit einem Auslassgraft.

Herzunterstützungspumpen, wie beispielsweise Ventricular Assist Devices (VAD), sind im Stand der Technik bekannt. Zudem existieren zahlreiche Vorschläge, wie derartige Pumpen mit einem Organ, wie beispielsweise mit einem Herzen, verbunden werden können.

Eine Vielzahl der VAD-Konnektorsysteme umfasst zum einen einen Konnektor, der beispielsweise an einem Apex eines linken Ventrikels, beispielsweise mit Hilfe einer Naht, verankert wird. Anschließend wird in den Ventrikel eine Öffnung geschnitten, durch welche die Blutpumpe in den Ventrikel geschoben werden kann oder eine Kanüle am Konnektor befestigt wird, welche mit ihrem nicht am Herzen befestigten Ende mit einem Konnektor der Blutpumpe verbunden wird.

Um eine sichere Befestigung zwischen dem Auslass der Blutpumpe und einem Blutgefäß, wie beispielsweise der Aorta oder der Subclavia, zu erreichen, sind im Stand der Technik oftmals im Wesentlichen starre Kanülen vorgesehen.

Die starren Kanülen weisen für das Blut sehr gute Fließeigenschaften auf. Allerdings sind die Kanülen aufgrund ihrer Steifigkeit nur schwer mit der Pumpe und dem Blutgefäß verbindbar, sofern der Thorax nicht großflächig geöffnet wird und die Kanüle unter Sichtkontrolle eines Arztes eingesetzt werden kann.

Um hier eine bessere Verarbeitbarkeit, auch für den Fall von minimalinvasiven Implantationstechniken, d. h. von Implantationstechniken, welche keine vollflächige Öffnung des Thorax benötigen, zu gewährleisten, wird vermehrt auf sogenannte Graftmaterialien zurückgegriffen. Diese Graftmaterialien können beispielsweise eine fluiddichte Textilschicht umfassen, welche ein Lumen definiert, durch welches das Blut geführt wird.

Nachteil dieser Grafts ist jedoch ihre niedrige Steifigkeit, wobei es leicht zu einer Torsion, einem Knicken oder einer Verengung des Graftlumens und somit einem reduzierten Blutfluss kommen kann.

Aufgabe der vorliegenden Anmeldung ist es, hier Abhilfe zu schaffen.

Die Aufgabe wird gelöst gemäß einem Auslassgraft nach Anspruch 1 sowie nach den Systemen und Verwendungen gemäß der nebengeordneten Ansprüche.

Der Auslassgraft umfasst in einer Ausführungsform ein erstes Ende und ein diesem gegenüberliegendes Ende, wobei das erste Ende mit einer Blutpumpe, wie beispielsweise einer Axial- oder Radialpumpe, verbindbar ist und das gegenüberliegende Ende mit einem Blutgefäß, wie beispielsweise der Aorta oder einer Subclavia, verbindbar ist. Zwischen diesen beiden Enden ist ein Lumen mit einer geschlossenen Umhüllung ausgebildet, wobei sich das Lumen im Wesentlichen entlang einer Längsachse erstreckt. Die Umhüllung des Auslassgrafts ist dabei vorzugsweise flexibel.

Die Umhüllung umfasst einen ersten, parallel zur Längsachse verlaufenden Streifen, wobei der erste Streifen aus einem Material besteht, das unter Röntgenbestrahlung konstrastreicher als die Umhüllung ist. Alternativ kann das Material des ersten Streifens auch unter anderen bildgebenden Verfahren kontrastreicher als das der Umhüllung sein; andere derartige bildgebende Verfahren sind beispielsweise ein Ultraschall- oder ein kernspintomografisches Verfahren.

Bei der Implantation eines derartigen Auslassgrafts kann anhand des ersten, parallel zur Längsachse verlaufenden Streifens mittels einer bildgebenden Quelle, wie beispielsweise einer Röntgenquelle, festgestellt werden, ob das erste Ende, welches mit einer Blutpumpe befestigt werden soll, und das diesem gegenüberliegende Ende, welches mit dem Blutgefäß verbunden werden soll, verdreht ist. Da eine Verdrillung bzw. Verdrehung oder Torsion des Auslassgrafts verhindert werden soll, kann nach der Befestigung des Auslassgrafts beispielsweise mit dem Blutgefäß auf einem Bildschirm betrachtet werden, ob der erste Streifen im Wesentlichen lediglich dem Verlauf der Längsachse folgt. Ist auf dem Bildschirm jedoch eine Verdrillung bzw. Verdrehung des Auslassgrafts zu erkennen, kann dieser derart orientiert werden, dass er vor dem Anschließen an die Blutpumpe (bzw. dem dieser gegenüberliegenden Ende) entdrillt werden kann und so ein für den Auslass der Blutpumpe zufriedenstellender großvolumiger und faltenfreier Auslass zur Verfügung gestellt wird. Auf diese Weise wird die Pumpleistung der angeschlossenen Blutpumpe verbessert.

Weitere Ausführungsformen sind in den Unteransprüchen skizziert.

In einer weiteren Ausführungsform umfasst die Umhüllung einen zweiten, parallel zur Längsachse verlaufenden Streifen aus einem Material, das unter Röntgenstrahlung kontrastreicher ist als die Umhüllung. Dabei ist der zweite Streifen vom ersten Streifen beabstandet. Bevorzugt ist die Beabstandung derart gewählt, dass der Abstand ein Winkelsegment von genau 180° oder von weniger als 180°, vorzugsweise weniger als 90°, jedoch mehr als 10° beträgt. Auf diese Art und Weise können der erste und zweite Streifen auf einem Bildschirm unter Röntgenbestrahlung einfach als voneinander getrennt wahrgenommen werden, und Verdrillungen bzw. Verdrehungen lassen sich leichter bemerken.

In einer weiteren Ausführungsform umfasst die Umhüllung einen dritten, die Umhüllung in Richtung der Längsachse helixartig umlaufenden Streifen aus einem Material, das unter Röntgenstrahlung kontrastreicher ist als die Umhüllung. Da die Helix eine vorbestimmte Steigung besitzt, kann bei einer Verdrillung bzw. Verdrehung des Auslassgrafts eine Änderung der Steigung beobachtet werden. Die Änderung der Steigung kann als Indiz bzw. Maß für eine Verdrillung bzw. Verdrehung und die Entdrillung herangezogen werden.

Die Helix kann dabei die Umhüllung im oder gegen den Uhrzeigersinn umlaufen. Ob der Auslassgraft bzw. der dritte Streifen die Umhüllung im oder gegen den Uhrzeigersinn umläuft, kann davon abhängig gemacht werden, ob der Auslassgraft mit einer Blutpumpe verbunden wird, welche im linken oder im rechten Ventrikel eingesetzt worden ist.

In einer weiteren Variante kann das Material der Umhüllung des Auslassgrafts einen polymeren Werkstoff aufweisen bzw. aus einem polymeren Werkstoff bestehen, in speziellen Ausführungsbeispielen aus PTFE. Weitere polymere Werkstoffe sind beispielsweise PE, PP, PET, PVC, PC, PMMA, PUR, Polysiloxane, PEEK, PSU oder PHEMA sowie Mischungen der vorgenannten polymeren Werkstoffe.

In einer weiteren Variante wird der Auslassgraft derart ausgebildet, dass die Umhüllung durch einen Kunststoffstreifen helixartig umlaufen und verstärkt wird. Dabei kann der Kunststoffstreifen eine sehr geringe Steigung aufweisen, so dass zwei benachbarte Umläufe entlang der Längsachse beispielsweise weniger als 0,5 cm, vorzugsweise weniger als 0,3 cm, voneinander entfernt sind. Die Verwendung des Kunststoffstreifens verstärkt den Graft und macht diesen formstabiler gegenüber einem externen Druck.

In einer weiteren Ausführungsform ist das erste Ende des Auslassgrafts mit einem Konnektor zur Verbindung mit der Blutpumpe versehen. Dabei kann der Konnektor einen sich in Richtung des gegenüberliegenden Endes des Auslassgrafts erstreckenden Fortsatz aufweisen. Der Fortsatz ist dabei aus einem Material gewählt, welches unter Röntgenbestrahlung einen gegenüber dem Material der Umhüllung größeren Kontrast besitzt. Auf diese Weise kann festgestellt werden, ob der Auslassgraft bzw. dessen Umhüllung gegenüber dem Konnektor verdrillt ist. In weiteren Ausführungsformen kann der Konnektor mehr als einen Fortsatz, beispielsweise zwei voneinander beabstandete Fortsätze, aufweisen. Der Fortsatz ist vorzugsweise derart ausgebildet, dass dieser mit dem ersten Streifen überlappt und eine Fortsetzung des Streifens bildet. Der Fortsatz kann in einigen Ausführungsformen beispielsweise streifenförmig ausgebildet sein. Auf diese Weise lassen sich Abweichungen bzw. Verdrillungen bzw. Verdrehungen zwischen dem ersten Streifen und dem Fortsatz auf einfache Art und Weise bestimmen. So kann der Fortsatz als Referenz für eine Verdrillung bzw. Verdrehung der Umhüllung herangezogen werden.

In einer weiteren Ausführungsform umfasst der Auslassgraft ein die Umhüllung zumindest teilweise einhüllendes Schutzelement. Dabei ist in einer Variante vorgesehen, dass sich das Schutzelement vom ersten Ende, welches mit der Blutpumpe verbindbar bzw. mit dieser verbunden ist, hin zum gegenüberliegenden Ende, jedoch nicht über den gesamten Umfang der Umhüllung hinweg erstreckt. Das heißt, das Schutzelement umgibt lediglich einen Teilabschnitt vom ersten Ende bis zu einem Punkt, welcher vom gegenüberliegenden Ende beabstandet ist. Mittels des einhüllenden Schutzelements wird eine Verstärkung des Grafts in der Nähe der Pumpe erzeugt.

Dabei kann in einer Ausführungsvariante vorgesehen sein, dass das Schutzelement eine unter Röntgenstrahlung sichtbare Markierung umfasst. Die sichtbare Markierung kann auch wiederum als Referenz für eine Verdrillung bzw. Verdrehung der Umhüllung dienen. In einer Variante ist die sichtbare Markierung parallel zur Längsachse der Umhüllung ausgerichtet und vorzugsweise streifenförmig. Alternativ kann die sichtbare Markierung helixartig, koaxial zum ersten Streifen der Umhüllung oder vom ersten Streifen beabstandet linienförmig ausgebildet sein. Hierdurch wird eine Verdrillung bzw. Verdrehung auf einfache Art und Weise erkannt.

In einer weiteren Ausführungsform umfasst die Umhüllung ein Endo- und/oder Exoskelett, vorzugsweise aus einem erinnerungsfähigen Material, wie beispielsweise Nitinol. Das Endo- bzw. Exoskelett bietet dabei eine zusätzliche Versteifung des Auslassgrafts. Auch können bestimmte Abschnitte des Auslassgrafts mit einer Form vorgeprägt werden, das heißt, das erinnerungsfähige Material kann beispielsweise Biegungen vordefinieren, so dass eine Verdrillung bzw. Verdrehung auf besonders zuverlässige Art und Weise vermieden werden kann. Dabei kann die Umhüllung das Endoskelett umhüllen oder in das Exoskelett eingebettet sein.

Vorzugsweise wird der Auslassgraft in Verbindung mit einer Blutpumpe, wie einer Axial- oder Radialpumpe, eingesetzt.

Ein derartiges System ist beispielsweise dazu geeignet, bei einer Implantation der Blutpumpe, insbesondere bei einer verdeckten Implantation der Blutpumpe im Rahmen einer minimal invasiven Implantation, eine Verdrillung bzw. Verdrehung des Auslassgrafts zu vermeiden und so eine verbesserte Pumpleistung herbeiführen zu können.

Nachfolgend wird der Auslassgraft anhand einiger Ausführungsbeispiele näher beschrieben.

Es zeigt
- Fig. 1: ein schematisches System gemäß dieser Anmeldung;
- Fig. 2: ein weiteres schematisches System gemäß dieser Anmeldung;
- Fign. 3a bis 3c: Ausführungsformen eines Auslassgrafts;
- Fign. 4a und 4b: weitere Ausführungsformen eines Auslassgrafts;
- Fign. 5a und 5b: weitere Ausführungsformen eines Auslassgrafts;
- Fign. 6a und 6b: Ausführungsformen eines Auslassgrafts mit Schutzelement;
- Fign. 7a bis 7c: einen Auslassgraft mit einem Konnektor;
- Fgn. 8a bis 8d: einen Auslassgraft mit einem Endo- bzw. Exoskelett;
- Fig. 9: eine schematische Darstellung der Verwendung eines Auslassgrafts bei einer Implantation.

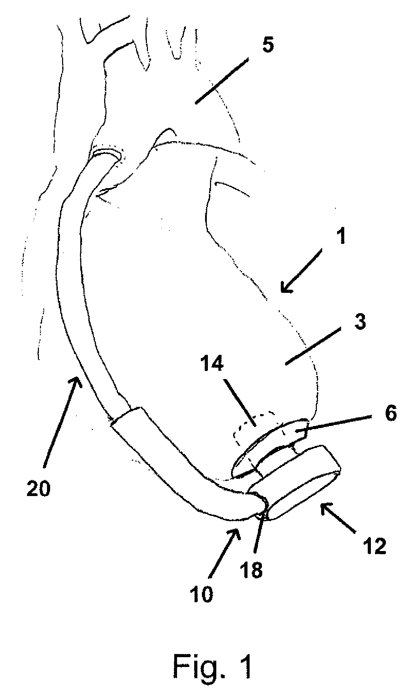

Figur 1 zeigt eine schematische Darstellung eines Herzens 1 sowie dessen linkes Ventrikel 3 und Aorta 5. In dem Herzen wurde ein ventrikuläres Assistenzsystem (VAD) 10 eingesetzt, welches unter anderem eine Blutpumpe 12 umfasst.

Die Blutpumpe 12 ist mit ihrem Einlass 14 in den Apex des linken Ventrikels 3 eingeführt. Die Blutpumpe 12 wird mittels eines Nahtrings 16 am Herzen fixiert. Die Blutpumpe 12 umfasst ferner einen Auslass 18, welcher mittels eines Auslassgrafts 20 an die Aorta 5 geführt wird, wobei das dem Auslass 18 gegenüberliegende Ende des Auslassgrafts 20 mit der Aorta vernäht ist, so dass Blut vom linken Ventrikel, unter Umgehung der Aortenklappe, in die Aorta 5 geführt werden kann. Die Blutpumpe 12 und der Auslassgraft 20 bilden zusammen ein System, welches beispielsweise bei der minimalinvasiven Implantation des VADs 10 eingesetzt werden kann. In den nachfolgenden Figuren sollen unter anderem Aspekte verschiedener anmeldungsgemäßer Auslassgrafts erläutert werden.

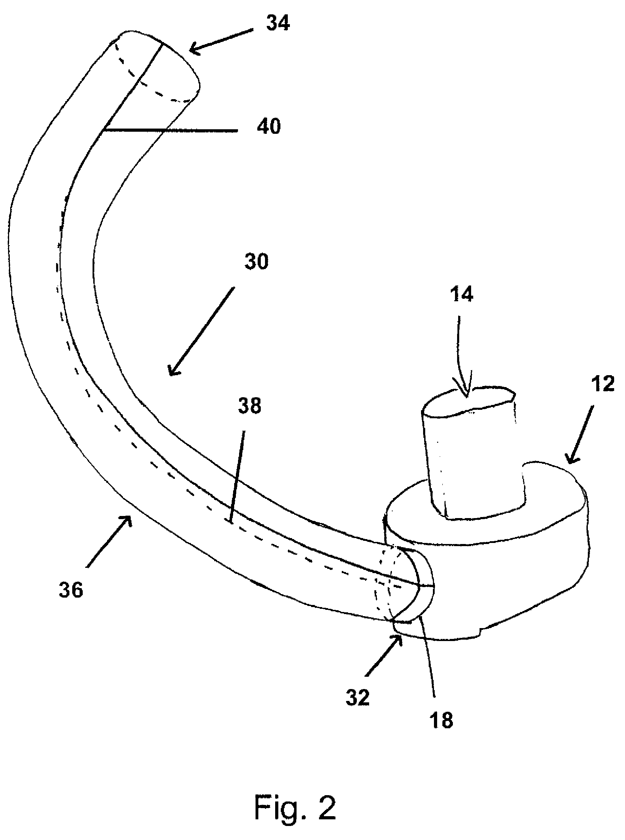

In Figur 2 ist ein schematischer Auslassgraft 30 dargestellt, welcher ein erstes Ende 32 umfasst, welches mit einem Auslass der Blutpumpe 12 verbunden ist. Dabei kann das erste Ende mittels eines Befestigungsmittels, wie beispielsweise einer Schelle, einem Kabelbinder oder einem nicht näher dargestellten Konnektor, an der Blutpumpe fixiert werden. Dem ersten Ende 32 gegenüberliegend befindet sich ein weiteres Ende 34, welches mit dem Blutgefäß, wie beispielsweise der Aorta oder der Subclavia, verbunden werden kann. Der Auslassgraft 30 kann direkt mit der Aorta vernäht werden, so dass Blut durch den Auslassgraft 30 in die Aorta strömen kann. Der Auslassgraft 30 besitzt eine Schlauchform 36, wobei der Schlauch ein zwischen dem ersten und zweiten Ende ausgebildetes Lumen begrenzt. Die das Lumen umgrenzende Umhüllung kann beispielsweise aus einem Kunststoff wie PTFE geformt sein. Weiterhin sind auch beschichtete Textilien, welche ein biokompatibles Einwachsen des Grafts ermöglichen, vorgesehen.

Auf einer Außenseite der Umhüllung befindet sich ein parallel zur Längsachse 38 des Schlauchs 36 verlaufender erster Streifen 40. Während das Material des Schlauchs 36 auf einem Röntgenbild nur wenig Kontrast erzeugt, kann der erste Streifen 40 beispielsweise aus einem Metall gefertigt sein, welches unter Röntgenbestrahlung einen hohen Kontrast erzeugt. Auf diese Weise kann auch bei einem Einsetzen des Grafts im Rahmen einer minimalinvasiven Operation, d. h. bei einer Operation, bei welcher ein direkter Sichtkontakt auf den bereits im Körper liegenden Graft nicht möglich ist, die Orientierung des Auslassgrafts mittels Röntgenbestrahlung des Thorax sichtbar gemacht werden. Da der Streifen unverdrillt parallel zur Längsachse verläuft, können Verdrillungen, d. h. eine Verdrehung des ersten Endes 32 gegenüber dem zweiten Ende 34 um die Längsachse 38 herum, schnell identifiziert werden. Die Verdrillungen bzw. Verdrehungen werden dabei z. B. als Kurvenform des ersten Streifens 40 sichtbar. Um ein besonders großes Volumen, welches für die Blutströmung zur Verfügung steht, zu schaffen, ist ein verdrillungsfrei sowohl mit der Pumpe als auch mit dem Blutgefäß verbundener Auslassgraft von Vorteil. Mittels des ersten Streifens 40 und dessen Beobachtung unter Röntgenbestrahlung kann das verdrillungsfreie Einsetzen des Grafts bei einer minimalinvasiven Operation gewährleistet werden.

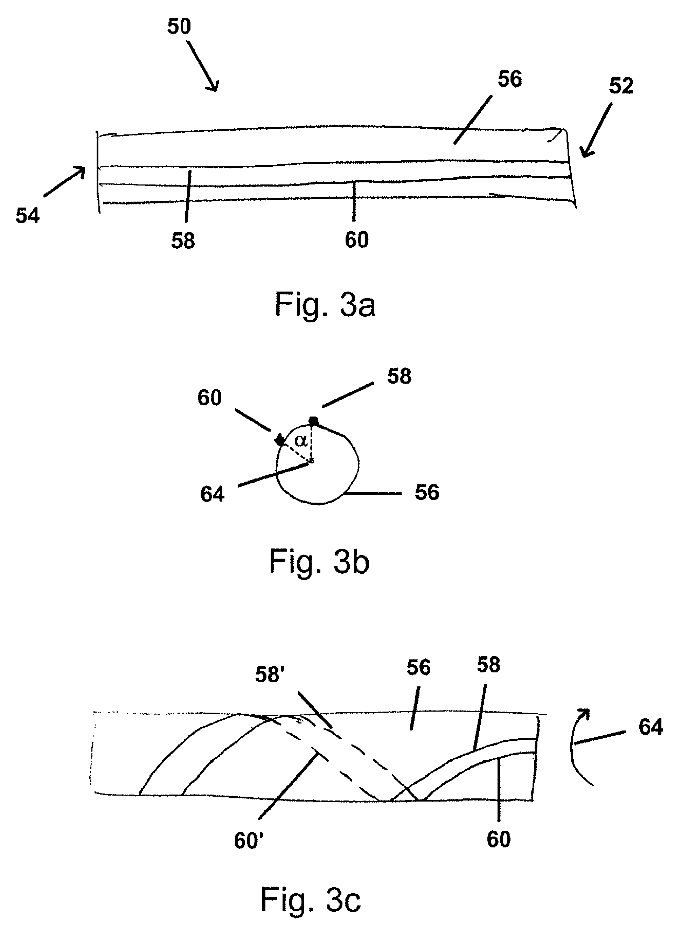

In den Figuren 3a bis 3c ist eine weitere Ausführungsform eines Auslassgrafts dargestellt. Der Auslassgraft 50 erstreckt sich zwischen dem ersten Ende 52 und einem zweiten Ende 54. Auf der Umhüllung 56 sind ein erster, parallel zur Längsachse verlaufender Streifen 58 und ein weiterer, zweiter Streifen 60 aufgebracht, wobei beide Streifen aus dem gleichen Material bestehen. In anderen Varianten können die Streifen aus verschiedenen Materialien bestehen. Die beiden Streifen sind parallel zueinander und voneinander beabstandet. Wie in Figur 3b zu erkennen ist, weist der erste Streifen 58 vom zweiten Streifen 60 eine Winkelbeabstandung von ungefähr α = 45° auf. In einem Röntgenbild bzw. einem Computertomografiebild werden die ersten und zweiten Streifen 58 bzw. 60 im unverdrillten Zustand, wie dieser beispielsweise in Figur 3a dargestellt ist, als parallel zueinander verlaufende Linien sichtbar. Wird nun das erste Ende gegenüber dem zweiten Ende in einer Drehrichtung 64 verdrillt, werden auch die (fest mit der Umhüllung 56) verbundenen Streifen 58 und 60 entsprechend verdreht. Dabei werden die parallel zueinander verlaufenden Linien in Form einer Kurve sichtbar. Die durchgehend eingezeichneten Abschnitte der Streifen 58 und 60 in Figur 3c sind dabei auf der Oberseite der Umhüllung liegende Abschnitte, die gestrichelt eingezeichneten Abschnitte 58' und 60' liegen, aus der Perspektive der Figur 3c betrachtet, auf der Rückseite der Umhüllung 56. Anhand der Veränderung des Verlaufs der Streifen ist während eines bildgebenden Verfahrens erkennbar, dass der Auslassgraft 50 verdreht ist. Dabei kann der Winkelabstand zwischen dem ersten und zweiten Streifen 58 und 60 frei gewählt werden. Es sind jedoch Abstände von weniger als 180° bevorzugt, um eine Rotationssymmetrie im Querschnitt des Auslassgrafts zu brechen.

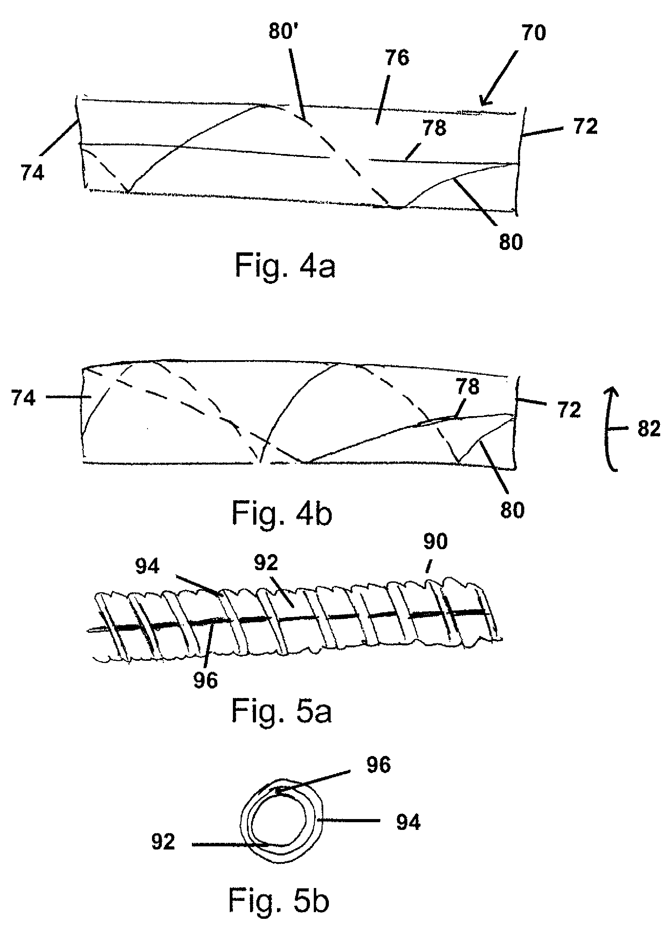

Eine weitere Variante eines Auslassgrafts ist in den Figuren 4a und 4b dargestellt. Der Auslassgraft 70 umfasst ein erstes Ende 72 und ein zweites Ende 74 sowie eine Umhüllung 76. In dem gezeigten Ausführungsbeispiel umfasst die Umhüllung einen ersten Streifen 78, welcher sich parallel zur Längsachse des Auslassgrafts 70 erstreckt. Ferner ist ein zweiter Streifen 80 aufgetragen, welcher sich helixartig um die Umhüllung 78 windet, was anhand der durchgezogenen Linie und des mit 80' dargestellten gestrichelten Abschnitts illustriert wird. Dabei verläuft der helixartig um die Umhüllung laufende Streifen 80 vom ersten zum zweiten Ende hin betrachtet im Uhrzeigersinn. Bei einer Verdrillung bzw. Verdrehung des ersten Endes 72 gegenüber dem zweiten Ende 74 in der Richtung 82 ändert sich der Verlauf des ersten Streifens 78 und des zweiten Streifens 80, so dass aus der unterschiedlichen Geometrie der Streifenverläufe eine Verdrillung bzw. Verdrehung auch im Rahmen eines bildgebenden Verfahrens sichtbar wird.

In den Figuren 5a und 5b ist eine weitere Variante eines Auslassgrafts dargestellt. Der Auslassgraft 90 ist hier lediglich abschnittsweise dargestellt. Das heißt, das erste und zweite Ende sind nicht sichtbar. Der Graft besitzt dabei eine Umhüllung 92, welche durch einen sich spiralförmig um die Umhüllung windenden Kunststoffstreifen 94 verstärkt wird. Ferner ist ein erster Streifen 96 sichtbar, welcher einen höheren Kontrast als die Umhüllung und als der Kunststoffstreifen aufweist. Das heißt, obgleich der Streifen 96 im sichtbaren Licht durch den Kunststoffstreifen 94 teilweise verdeckt wird, ist der erste Streifen bei einem bildgebenden Verfahren, wie beispielsweise einer Computertomografie, weiterhin als durchgehende Linie zu erkennen. Anhand der Figur 5b ist dies nochmals illustriert.

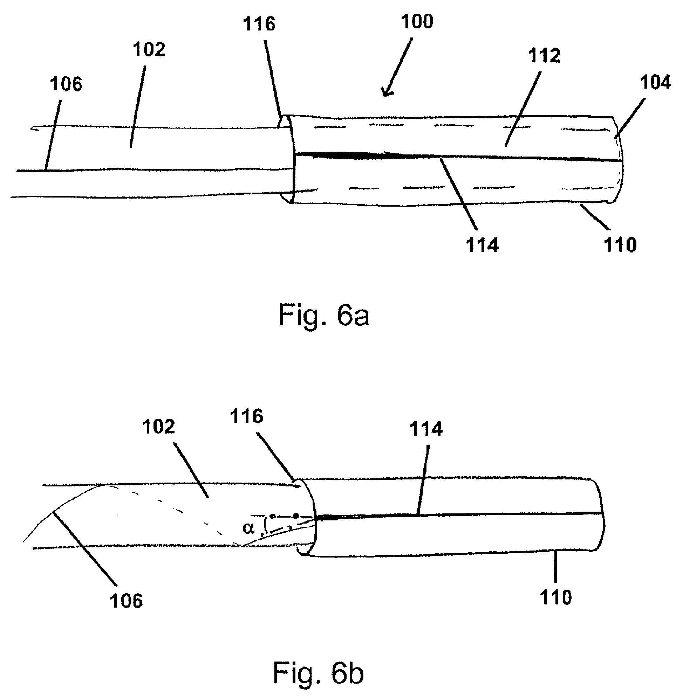

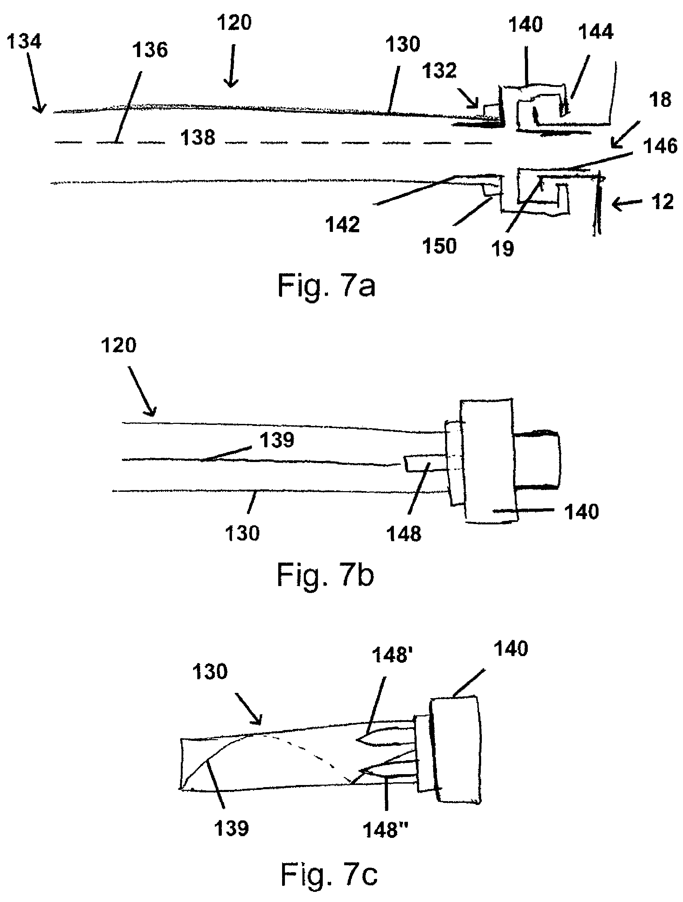

In den Figuren 6a und 6b ist eine weitere Variante eines Auslassgrafts dargestellt. Der Auslassgraft 100 umfasst eine ein Lumen umschließende Umhüllung 102 mit einem ersten Ende 104 und einem nicht dargestellten zweiten Ende. Auf der Umhüllung befindet sich abschnittsweise ein erster Streifen 106, welcher im Wesentlichen parallel zur Längsachse der Umhüllung verläuft. Vom ersten Ende 104 erstreckt sich in Richtung des gegenüberliegenden Endes ein Schutzelement 110, welches beispielsweise aus einem biokompatiblen Kunststoff oder einem Metall bestehen kann. Das Schutzelement weist neben einer die Umhüllung umschließenden Ummantelung 112 einen Streifen 114 auf, welcher gegenüber der Ummantelung 112 einen unter Röntgenstrahlung größeren Kontrast besitzt. Anhand der Figur 6a ist erkennbar, dass der Streifen 114 im Wesentlichen auch parallel zur Längsachse der Umhüllung 102 verläuft. Jedoch ist der Streifen 106 im unverdrillten Zustand gegenüber dem Streifen 114 versetzt. Wird nun, wie in Figur 7b dargestellt, die Umhüllung 102 von ihrem ersten Ende zum zweiten Ende hin verdreht, wirkt sich dies nicht auf das Schutzelement 110 aus, deren Streifen 114 weiter im Wesentlichen parallel zur unverdrillten Längsachse der Umhüllung 102 verläuft. Der erste Streifen 106 jedoch ist verdrillt und bildet beispielsweise am Ende 116 des Schutzelements 110 mit dem ersten Streifen 106 einen Winkel α, welcher größer als 0 ist. Das Winkelmaß kann beispielsweise als Maß für den Grad der Verdrillung bzw. Verdrehung verwendet werden.

Eine weitere Variante eines Auslassgrafts ist in den Fign. 7a bis 7c dargestellt. Der Auslassgraft 120 umfasst eine Umhüllung 130, welche ein erstes Ende 132 und ein diesem gegenüberliegendes zweites Ende 134 und ein sich entlang einer Längsachse 136 erstreckendes Lumen 138 umfasst. Der Auslassgraft 120 umfasst ferner einen Konnektor 140, welcher beispielsweise aus Titan oder einem anderen biokompatiblen Metall gefertigt sein kann. Weitere Metalle können Co-Legierungen, Ti-Legierungen (α + β), Ni-Legierungen, Cr-Legierungen, Ta-Legierungen oder Pt-Legierungen sein. Diese metallischen Werkstoffe können in manchen Ausführungsformen auch als Werkstoffe für an anderen Stellen dieser Anmeldung erwähnte Metalle herangezogen werden. Alternativ oder in Kombination zu den metallischen Werkstoffen können auch keramische Werkstoffe wie Al₂O₃, ZrO₂ oder TiO₂ eingesetzt werden. Der Konnektor 140 zeigt im dargestellten Querschnitt einen ersten Rohrabschnitt 142, welcher sich vom ersten Ende zum zweiten Ende der Umhüllung 130 hin erstreckt und aufweichen das erste Ende 132 der Umhüllung gesteckt werden kann. Ferner umfasst der Konnektor einen Klauenring 144, welcher einen Kragen 19 des Auslasses 18 der Blutpumpe 12 hintergreifen kann. Weiterhin besitzt der Konnektor 140 einen zweiten Rohrabschnitt 146, welcher in den Auslass 18 eingeführt werden kann, so dass eine fluiddichte Verbindung zwischen der Umhüllung und der Blutpumpe 12 hergestellt werden kann. Der erste und zweite Rohrabschnitt sind jeweils optional.

Die Umhüllung 130 kann beispielsweise mittels eines Kabelbinders 150 mit dem Konnektor verbunden werden. Dabei kann der Konnektor bereits vor der Implantation des Auslassgrafts mit der Umhüllung verbunden werden, damit die Verbindung des Auslassgrafts 120 mit der Blutpumpe 12 vereinfacht wird. In Figur 7a ist der Auslassgraft 120 derart dargestellt, wie er unter einem Röntgenbild zu sehen wäre. Es ist eine Außengrenze der Umhüllung zu erkennen. Ferner ist der erste Streifen 139, welcher parallel zur Längsachse 136 verläuft, zu erkennen. Der Konnektor 140 umfasst neben den bereits beschriebenen Merkmalen ferner einen Fortsatz 148, welcher sich im Wesentlichen parallel zur Längsachse der Umhüllung erstreckt. Dabei überlappen sich der Fortsatz 148 und der erste Streifen 139. Auch hier ist eine Verdrillung bzw. Verdrehung des Auslassgrafts vom ersten zum zweiten Ende der Umhüllung hin einfach in der Röntgenaufnahme sichtbar. Obgleich in Figur 7b lediglich ein rechteckiger Fortsatz 148 dargestellt ist, kann ein alternativer Konnektor, wie er beispielsweise in Figur 7c dargestellt wird, mehrere Fortsätze 148' und 148" umfassen. In Figur 7c ist zudem eine Verdrillung bzw. Verdrehung der Umhüllung 130 dargestellt, wobei erkennbar ist, dass der erste Streifen 139 seine "unverdrillte" Position zwischen den Fortsätzen 148' und 148" verlässt. Die Fortsätze können zudem dabei helfen, festzustellen, ob der Konnektor gegenüber der Umhüllung fest fixiert ist oder ob dieser durchrutschen kann, sofern vor der Implantation des Auslassgrafts eine feste Ausrichtung der Fortsätze 148 bzw. 148' und 148" gegenüber dem ersten Streifen 139 gewählt wurde. Obgleich in dem hier aufgeführten Beispiel Fortsätze 148 und 148" gezeigt sind (welche weiter in Richtung des zweiten Endes der Umhüllung 130 ragen als der erste Rohrabschnitt 142), kann in einer alternativen Ausführungsform der Rohrabschnitt 142 beispielsweise geschlitzt sein, so dass der Schlitz im Metall lediglich einen geringen Röntgenkontrast aufweist. Auch kann eine feste Zuordnung zueinander auf dem Röntgenbild leicht erkannt werden.

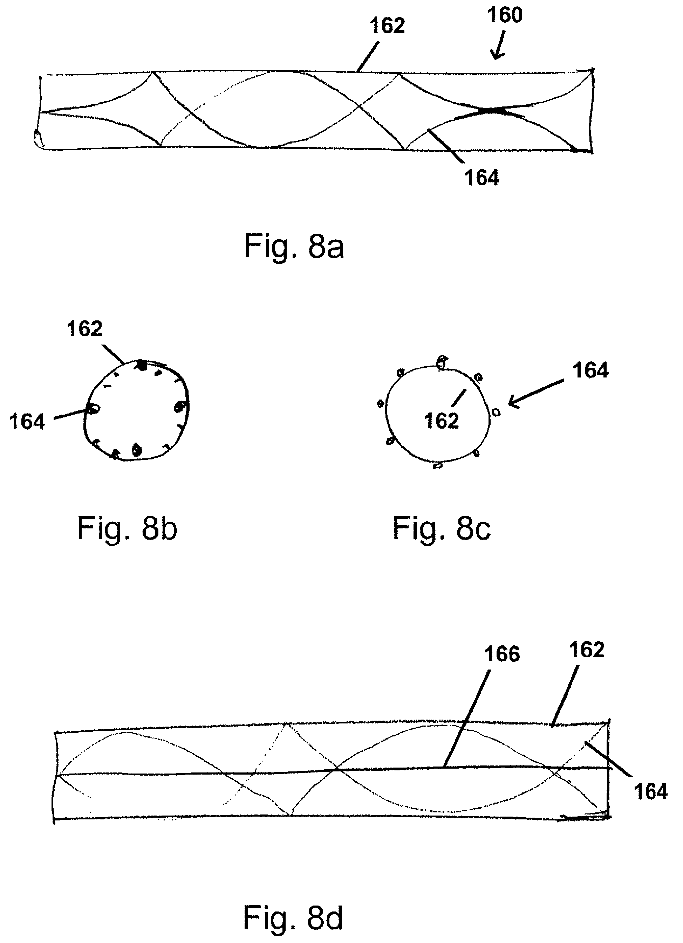

In den Figuren 8a bis 8d ist eine weitere Variante eines Auslassgrafts 160 dargestellt. Der Auslassgraft 160 umfasst eine Umhüllung 162 sowie eine aus Nitinol gefertigte Struktur 164, welche, wie in Figur 8b dargestellt, als Endoskelett die Umhüllung 162 aufspannt. Alternativ könnte die Nitinolstruktur 164 auch als Exoskelett ausgeführt sein, wie in Figur 8c dargestellt. Da Nitinol beispielsweise im Röntgenbild sichtbar ist, würde sich in Verbindung mit dem Streifen 166 ein Bild wie in Figur 8d gezeigt ergeben.

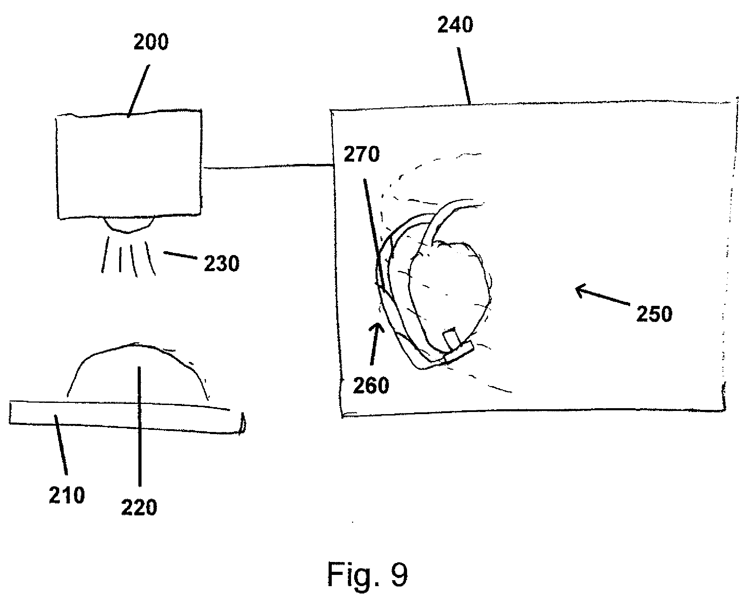

Anhand der Figur 9 soll noch kurz die Verwendung eines Auslassgrafts mit einem Röntgenkontraststreifen bei der minimalinvasiven Implantation eines Herzpumpensystems, wie eines VADs, erläutert werden. In Figur 9 ist ein Röntgentomografiegerät 200 dargestellt, welches einen auf einem Operationstisch 210 liegenden Patienten 220 mit Röntgenstrahlung 230 bestrahlt. Das sich ergebende und durch den Röntgentomografen berechnete Bild des Thorax wird auf einem Monitor 240 ausgegeben. Auf dem Monitor ist das Bild 250 zu sehen, welches hier einen schematisch dargestellten Brustkorb und ein darunter liegendes Herz mit einem implantierten VAD zeigt. Am Graft 260 ist deutlich erkennbar, dass der erste Streifen mehrfach verdrillt ist. Um die Verdrillung bzw. Verdrehung aufzulösen, müsste beispielsweise das mit der Pumpe verbundene Ende des Auslassgrafts im Uhrzeigersinn rotiert werden, um eine Entdrillung zu bewirken. Um dies festzustellen, ist kein direkter Sichtkontakt mit dem Graft nötig, sondern es kann allein der Röntgenkontrast des Streifens 270 genutzt werden.

Obgleich in sämtlichen dargestellten Ausführungsbeispielen ein erster parallel zur Längsachse der Umhüllung verlaufender Streifen erläutert wurde, ist in anderen Ausführungsformen ein parallel zur Längsachse verlaufender Streifen nicht notwendig. So kann beispielsweise ein beliebig geformter Streifen mit höherem Kontrast als das Material der Umhüllung eingesetzt werden. Beliebige Formen, beispielsweise sich mäanderförmig erstreckende Röntgenkontraststreifen, helixartig geformte Kontraststreifen, punktförmige, kreisförmige oder kreuzförmige Kontraststreifen, umlaufende, parallel entlang der Längsachse des Grafts zueinander angeordnete, ringförmige Kontraststreifen oder andere Geometrien können in einigen Ausführungsbeispielen - alter-nativ oder zusätzlich zu einem Kontraststreifen gemäß der Ansprüche-eingesetzt werden.

## Patentansprüche

1. Auslassgraft (30) für eine Blutpumpe, wobei ein erstes Ende (32) des Auslassgrafts mit einer Blutpumpe (12) und ein dem ersten Ende gegenüberliegendes Ende (34) des Auslassgrafts mit einem Blutgefäß verbindbar ist und zwischen den beiden Enden ein Lumen mit einer geschlossenen Umhüllung (36) und einer Längsachse ausgebildet ist,
wobei
die Umhüllung einen ersten parallel zur Längsachse verlaufenden Streifen (40) umfasst und der erste Streifen aus einem unter Röntgenstrahlung kontrastreicheren Material als die Umhüllung besteht,

2. Auslassgraft nach Anspruch 1, wobei die Umhüllung einen zweiten, parallel zur Längsachse verlaufenden Streifen aus einem unter Röntgenstrahlung kontrastreicheren Material als die Umhüllung umfasst und der zweite Streifen (58; 60) vom ersten Streifen beabstandet ist.

3. Auslassgraft nach einem der Ansprüche 1 oder 2, wobei die Umhüllung einen dritten, die Umhüllung helixartig umlaufenden Streifen (80) aus einem unter Röntgenstrahlung kontrastreicheren Material als die Umhüllung umfasst.

4. Auslassgraft nach Anspruch 3, wobei der dritte Streifen die Umhüllung vom ersten zum gegenüberliegenden Ende im Uhrzeigersinn umläuft.

5. Auslassgraft nach Anspruch 3, wobei der dritte Streifen die Umhüllung vom ersten zum gegenüberliegenden Ende gegen den Uhrzeigersinn umläuft.

6. Auslassgraft nach einem der vorhergehenden Ansprüche, wobei die Umhüllung einen polymeren Werkstoff umfasst und vorzugsweise aus einem polymeren Werkstoff und dabei besonders vorzugsweise aus PTFE besteht.

7. Auslassgraft nach einem der vorhergehenden Ansprüche, wobei die Umhüllung durch einen Kunststoffstreifen (94) helixartig umlaufen und verstärkt wird.

8. Auslassgraft nach einem der vorhergehenden Ansprüche, wobei das erste Ende einen Konnektor (140) zur Verbindung mit der Blutpumpe umfasst oder mit diesem verbindbar ist und der Konnektor einen sich in Richtung des gegenüberliegenden Endes des Auslassgrafts erstreckenden Fortsatz (148; 148'; 148") aufweist.

9. Auslassgraft nach Anspruch 8, wobei der Fortsatz den ersten Streifen überlappt und der Fortsatz vorzugsweise streifenförmig und koaxial zum ersten Streifen verläuft.

10. Auslassgraft nach einem der vorhergehenden Ansprüche, wobei das erste Ende ein die Umhüllung zumindest teilweise einhüllendes Schutzelement (110) umfasst.

11. Auslassgraft nach Anspruch 10, wobei das Schutzelement eine parallel zur Längsachse ausgerichtete, unter Röntgenstrahlung sichtbare Markierung umfasst und die Markierung (114) vorzugsweise streifenfömig und parallel zum ersten Streifen verläuft.

12. Auslassgraft nach einem der vorhergehenden Ansprüche, wobei die Umhüllung ein Endo- und/oder Exoskelett (164), vorzugsweise aus einem erinnerungsfähigen Material, besitzt.

13. System, umfassend eine Blutpumpe und einen an einem Auslass der Blutpumpe angebundenen Auslassgraft nach einem der vorhergehenden Ansprüche.

14. Verwendung eines Auslassgrafts nach einem der Ansprüche 1 bis 11 bei der Implantation einer Blutpumpe, insbesondere bei einer verdeckten Implantation der Blutpumpe.
